# EUROPEAN PATENT SPECIFICATION

(11) **EP 4 512 380 B1**
(45) Date of publication and mention of the grant of the patent: **08.04.2026**
(21) Application number: 23799118.7
(22) Date of filing: 06.03.2023
(51) Int. Cl.: A61F 9/007

(54) **DELIVERY DEVICE FOR EYE IMPLANT**
FREISETZUNGSVORRICHTUNG FÜR AUGENIMPLANTAT
DISPOSITIF DE POSE POUR IMPLANT OCULAIRE

(30) Priority: 06.05.2022 CN 202210488553
(43) Date of publication of application: 26.02.2025
(73) Proprietor: Health Guard (Suzhou) Biomed. Technology Co., Ltd, Suzhou, Jiangsu 215123 (CN)
(72) Inventor: LIU, Hua, Suzhou, Jiangsu 215123 (CN); FAN, Zhongpeng, Suzhou, Jiangsu 215123 (CN)
(74) Representative: Barker Brettell LLP
(86) International application number: PCT/CN2023/079794
(87) International publication number: WO 2023/213129

(56) References cited:
- CN-A- 101 969 898
- CN-A- 105 899 170
- CN-A- 110 584 874
- CN-A- 111 643 269
- CN-A- 112 638 332
- CN-A- 114 848 289
- US-A1- 2011 009 874
- US-A1- 2013 253 437
- US-A1- 2016 158 049
- US-A1- 2016 296 739
- US-A1- 2018 221 182
- US-A1- 2019 321 075
- US-A1- 2020 015 960
- US-A1- 2020 390 601

## Description

### TECHNICAL FIELD

The present application relates to the technical field of medical devices, and in particular to an ocular implant delivery device.

### BACKGROUND

Glaucoma is a group of diseases that are commonly characterized by optic nerve atrophy and depression, visual field loss and decreased vision. Glaucoma is associated with increased intraocular pressure, usually due to the inability of the ocular drainage channels to adequately remove aqueous humor from the anterior chamber of the eye, or due to excessive aqueous humor production caused by the ciliary body in the eye. Patients often experience symptoms such as nausea and pain, if not treated in time, it will lead to vision loss. For the treatment of glaucoma, surgical filtration methods can be used to reduce intraocular pressure by creating a fluid flow path between the anterior chamber and the low-pressure area. Ocular implants can be positioned in the eye to drain fluid from the anterior chamber to multiple locations, such as the sub-Tenon's space, subconjunctival space, suprascleral vein, suprachoroidal space, Schlemm canal, and intrascleral space. Ocular implants are generally often prepared from a selection of animal-derived/non-animal-derived polymers, metals, and other materials, and are generally prevalent in tubular or tube-like form. According to the different mechanisms of reducing intraocular pressure in glaucoma, drainage of aqueous humor through the subconjunctival space and the superior scleral vein is more effective. The common surgical pathways are to penetrate the atrial angle area through the anterior chamber from a micro-corneal incision, reach the subconjunctival space through the sclera, or reach the suprachoroidal space through the anterior chamber angle. This type of operation requires a corresponding delivery mechanism to deliver the implant to the corresponding part of the eye.

CN105899170B discloses an inserter for treating glaucoma for positioning an intraocular shunt within an eye for treating glaucoma, which is used to position an intraocular shunt in the eye to treat glaucoma. The drive assembly of the inserter is a cylindrical member coupled to a needle via a needle recess and to a plunger via a plunger recess. The drive assembly further includes a sliding recess that longitudinally overlaps the needle recess and plunger recess. The sliding groove is used to couple the sliding assembly to the drive assembly, and the cylindrical member is driven to rotate in the longitudinal direction by movement of the sliding assembly on the housing to move the needle and the plunger in the axial direction. The cylindrical member is provided with three sections of grooves, and the structural design is relatively complex. Firstly, because the rotational fit of the grooves of the cylindrical member in the longitudinal direction is too long, which makes the structure insufficiently compact and leads to low accuracy; secondly, the cylindrical member cannot be assembled in one piece in a process, and the cylindrical member needs to be processed in two halves and then spliced together, which further affects the pushing accuracy; thirdly, the friction surface of the structure is larger when moving, which results in a larger dissipation of the pushing force and a low pushing efficiency; thus, it is necessary to make improvements to the ocular implant delivery device in the existing art.

US2020/390601 A1 discloses a system for preparation of an implant and ab interno insertion of the implant into an eye. US2016/158049 A1 discloses a system for delivering a stent from a catheter sheath in a longitudinal direction. US2016/296739 A1 discloses a pellet delivery device. US2020/015960 A1 discloses an Intraocular lens (IOL) injector. US2018/221182 A1 discloses a system for delivering an implant.

### SUMMARY

The present invention relates to an ocular implant delivery device and is defined by the appended claims. The purpose of the present application is to solve at least some of the problems of the ocular implant delivery device in the existing art, including low precision and difficulties in being molded in one piece, thereby affecting the pushing precision when splicing as well as having a large friction surface when pushing, resulting in a large dissipation of pushing force and low pushing efficiency. Therefore, there is provided an ocular implant delivery device, which is capable of realizing the precise delivery of ocular implant to the subconjunctival cavity, thereby reducing the intraocular pressure value of the patient with high intraocular pressure, realizing the simple structural design, and having better doctor experience.

Embodiments of the present application provide an ocular implant delivery device including: a housing, a rotating wheel, a push needle linkage assembly, a retracting linkage assembly, a sleeve, and a puncture needle. The housing is provided with a through hole at a distal end of the housing, and the sleeve extends an inside of the housing to an outside of the housing through the through hole, and is connected to the housing. The puncture needle runs through the sleeve, a proximal end of the puncture needle is fixedly connected to the retracting linkage assembly, and a push needle of the push needle linkage assembly runs through an inner hole of the puncture needle. The rotating wheel is connected to an inside of the housing via rotating shafts, an opening corresponding to the rotating wheel is provided on the housing, and the rotating wheel is drivingly connected to the push needle linkage assembly and the retracting linkage assembly via a rack and pinion mechanism, so that the push needle linkage assembly is driven to move toward the distal end of the housing, or the retracting linkage assembly is driven to move away from the distal end of the housing by the rotating wheel rotating.

According to the invention, the rotating shafts disposed on two sides of the rotating wheel each are provided with a sector gear. The push needle linkage assembly and the retracting linkage assembly each are provided with a rack at a proximal end, the racks on the push needle linkage assembly and the retracting linkage assembly respectively extending to the two sides of the rotating wheel respectively, and each of the racks being configure to intermeshing with the sector gear on the rotating shaft on the corresponding side of the rotating wheel. With this arrangement, the needle push link assembly can be driven to move toward the distal end of the housing by the rotation of the rotating wheel, and the ocular implant can be accurately delivered to the subconjunctival cavity, thereby reducing the intraocular pressure value of patients with high intraocular pressure. After the implantation is completed, with continuing to rotate the rotating wheel, the retracting linkage assembly can drive the puncture needle to retract, achieving linear needle retraction and improving the safety of the operation.

In some embodiments, the sector gears on the rotating shafts on the two sides of the rotating wheel are disposed on the same side of the rotating shafts along a radial direction of the rotating shaft, and the racks disposed on the push needle linkage assembly and on the retracting linkage assembly are arranged at intervals along a radial direction of the rotating wheel, and each of the racks is configured to intermesh with the sector gear on the rotating shaft on the corresponding side. With this arrangement, the needle linkage assembly and the retracting linkage assembly are prevented from moving at the same time, thereby improving the safety of the operation.

In some embodiments, the first rack on the push needle linkage assembly is disposed above the rotating shafts of the rotating wheel, and the second rack on the retracting linkage assembly is disposed below the rotating shafts of the rotating wheel. The sector gear on the rotating shaft of the rotating wheel corresponding to the first rack on the push needle linkage assembly intermeshes with the first rack on the push needle linkage assembly in an initial state, and the sector gear on the rotating shaft of the rotating wheel corresponding to the rack on the retracting linkage assembly is located on the upper part of the rotating shaft in the initial state. With this arrangement, when the rotating wheel is rotated toward the distal end of the housing, the push needle linkage assembly can drive the push needle to move toward the distal end of the housing, while the retracting linkage assembly remains stationary. When the push needle linkage assembly completes the delivery of the ocular implant, the push needle linkage assembly no longer moves. When the rotating wheel continues to be rotated, it can drive the retracting link to move toward the proximal direction, thereby realizing the linear retraction of the puncture needle and improving the safety of the operation.

In some embodiments, the first rack on the push needle linkage assembly is disposed below the rotating shafts of the rotating wheel, the second rack on the retracting linkage assembly is disposed above the rotating shafts of the rotating wheel. The sector gear on the rotating shaft of the rotating wheel corresponding to the rack on the push needle linkage assembly intermeshes with the first rack on the push needle linkage assembly in an initial state, and the sector gear on the rotating shaft of the rotating wheel corresponding to the rack on the retracting linkage assembly is located on the lower part of the rotating shaft in the initial state. The principle of this scheme is exactly the same as the above scheme, except that when performing a delivery operation for ocular implant, the rotating wheel rotates toward the proximal end of the housing.

According to the invention, a central angle of the sector gear is in a range of 30° to 180°. This arrangement not only allows both the push needle linkage assembly and the retracting linkage assembly to have sufficient movable distance to ensure the smooth completion of the ocular implant operation, but also allows only one of the push needle linkage assembly and the retracting linkage assembly to be in a moving state to avoid the push needle linkage assembly and the retracting linkage assembly moving at the same time and affecting the operation.

In some embodiments, the push needle linkage assembly includes a push needle, a push needle link, and a first rack. A proximal end of the push needle is fixedly connected to a distal end of the push needle link, a proximal end of the push needle link is fixedly connected to the first rack horizontally arranged, and teeth on the first rack are disposed toward the rotating shaft. With this arrangement, the push needle linkage assembly can be driven to move toward the distal end by rotating the rotating wheel, so as to realize the delivery of the ocular implant to the surgical site of the patient's eye and ensure the smooth completion of the operation.

In some embodiments, the push needle linkage assembly further includes a folded corner portion, the folded corner portion being disposed at an end of the push needle link close to the first rack and fixedly connected to the first rack. The folded corner portion has an extending direction that deviates from an extending direction of the first rack, and extends from the first rack in a direction toward the retracting linkage assembly. With setting the folded corner portion, the distance between the push needle link and the retracting linkage assembly is reduced, the first rack can be arranged horizontally and the teeth on the first rack can be disposed toward the rotating shaft, and the push needle link can be arranged at the distal end of the rotating wheel, thereby making the internal structure of the ocular implant delivery device more compact.

In some embodiments, the retracting linkage assembly includes a connector, a retracting link, and a second rack horizontally arranged, the connector being fixedly connected to a distal end of the retracting link, the second rack being fixedly connected to the proximal end of the retracting link, and teeth on the second rack being disposed toward the rotating shaft. With this arrangement, the retracting linkage assembly can be driven to move toward the proximal direction by rotating the rotating wheel, and the puncture needle can be retracted after the implant is delivered, ensuring that the needle is retracted in a straight line during the needle retraction process, thereby improving the safety of the operation.

In some embodiments, the connector is provided with a through hole along an extending direction of the retracting link, and a push needle of the push needle linkage assembly extends the through hole and extends into the inner hole of the puncture needle. The through hole is provided to guide the push needle of the push needle linkage assembly, thereby improving the safety of the operation.

In some embodiments, along the extending direction of the retracting link, an inner diameter of the through hole is smaller than a maximum dimension of a push needle link of the push needle linkage assembly along the radial direction of the through hole. With this arrangement, the moving distance of the push needle linkage assembly is limited, thereby preventing the push needle linkage assembly from moving a distance exceeding the expected range, thereby improving the safety of the operation.

In some embodiments, the retracting linkage assembly further includes a folded corner portion which is disposed at an end of the retracting link close to the second rack and is fixedly connected to the second rack, and the folded corner portion has an extending direction that deviates from the second rack, and extends from the second rack in a direction toward the push needle linkage assembly. By setting the folded corner portion, the distance between the push needle link and the retracting linkage assembly is reduced, the second rack can be arranged horizontally, the teeth on the first rack can be arranged toward the rotating shaft, and the retracting link can be arranged at the distal end of the rotating wheel, thereby making the internal structures of the ocular implant delivery device more compact.

In some embodiments, the ocular implant delivery device further includes a stop seat configured to define a distance between the push needle linkage assembly and the retracting linkage assembly. By setting the stop seat to define the distance between the push needle linkage assembly and the retracting linkage assembly to enable them to form an integral structure, thereby improving space utilization.

In some embodiments, the stop seat comprises a first extending section and a second extending section intersecting each other. The first extending section is fixedly connected to the second extending section, and is fixedly connected to the retracting linkage assembly or the push needle linkage assembly, and extends from the retracting linkage assembly to the push needle linkage assembly by a predetermined distance. The second extending section extends from the first extending section in a direction facing away from the first extending section, and an orthographic projection of the second extending section falls within a range of the retracting linkage assembly and the push needle linkage assembly along an extending direction of the first extending section. Through this arrangement, the push needle linkage assembly or the retracting linkage assembly passes through the stop seat, so that the distance between the push needle linkage assembly and the retracting linkage assembly is defined to form an integral structure.

In some embodiments, a proximal end of the sleeve is fixedly connected to a sleeve seat which is disposed in the housing at a position close to the through hole, and the sleeve extends through the through hole on the housing to the outside of the housing. With this arrangement, the connection between the sleeve and the housing can be firmer, and the puncture needle can conveniently penetrate through the sleeve to puncture the position where the ocular implant needs to be conveyed.

In some embodiments, the puncture needle is provided with a puncture needle seat at a proximal end, the puncture needle being fixedly connected to the puncture needle seat and running through the sleeve, and the puncture needle seat being fixedly connected to a distal end of the retracting linkage assembly. This arrangement can make the connection between the sleeve and the housing more secure, and facilitate the puncture needle to pass through the sleeve and puncture the position where the ocular implant needs to be delivered.

In some embodiments, the housing includes an upper housing and a lower housing fixedly connected to the upper housing, the upper housing and the lower housing each comprises a cylinder portion, a gripping portion, and a conical portion, the cylinder portion, the gripping portion and the conical portion that are fixedly connected together. A rotating wheel mount is disposed on an inner wall surface of the cylinder portion of the housing, and the rotating wheel is fitted on the rotating wheel mount via the rotating shafts. A stop block is provided on an inner wall surface of at least one of the cylinder portion, the gripping portion, and the conical portion, and the stop block is configured to limit the push needle linkage assembly and the retracting linkage assembly in a radial direction. By providing the gripping portion, it is convenient for the doctor to hold the ocular implant delivery device for operation, and by providing the rotating wheel mount, it is convenient to assemble the rotating wheel and enable the rotating wheel to rotate flexibly. By providing the stop block in the housing, the push needle link and the retracting link can be radially limited to prevent them from radially deviating, thereby improving the accuracy of the operation.

In some embodiments, the rotating wheel is provided with teeth on an outer circumferential surface, and the housing is provided with an operating-sliding sleeve at a position corresponding to the opening, the operating-sliding sleeve being slidably fitted with the housing. The operating-sliding sleeve is provided with a rack portion on an inner wall, the rack portion intermeshing with the teeth on the rotating wheel. By providing the operating-sliding sleeve, it is convenient for the doctor to push the operating-sliding sleeve by hand to perform operation. Compared with the scheme of directly rotating the rotating wheel to perform operation, the flexibility and convenience of the operation of the ocular implant delivery device can be greatly improved, making the surgical process more labor-saving and more efficient.

In some embodiments, the housing is provided with a spring sheet on an outer wall, the spring sheet extending to the outside of the housing and cooperating with the rack portion on an inner wall of the operating-sliding sleeve. By providing the spring sheet, the doctor will have obvious tactile feedback when pushing the operating-sliding sleeve to perform operation. Every time the rotating wheel moves a tooth, the spring sheet will engage with the tooth, which can effectively prevent the rotating wheel from rotating in the opposite direction due to misoperation during operation, thereby improving the safety of the operation of the ocular implant delivery device. In addition, by providing the spring sheet, the doctor can avoid using too much force during operation and the rotating wheel rotating too fast, which will affect the quality of the operation, thereby further improving the safety of the operation.

It should be noted that the distal end refers to the end facing away from the doctor during ocular implant operation, and the proximal end refers to the end close to the doctor during ocular implant operation. It can be interpreted that the distal end refers to the end close to the patient's eye that needs operation, and the proximal end is the end facing away from the patient's eye.

The embodiments of the present application has at least some of the following positive effects:
1) The ocular implant delivery device can first drive the push needle linkage assembly to move toward the distal end of the housing through the rotation of the rotating wheel to realize the delivery of the ocular implant. After the ocular implant is delivered in place, when the rotating wheel continues to be rotated, the push needle linkage assembly no longer moves, and the retracting linkage assembly drives the puncture needle to retract the needle toward the proximal end of the housing. Through this setting, the structure of the ocular implant delivery device can be simplified. During the ocular implant delivery process, the push needle moves in a straight line to improve the accuracy of the operation. After the ocular implant delivery is completed, the puncture needle can be retracted in a straight line to improve the safety of the operation.
2) In the ocular implant delivery device, the structure of the internal driving assembly is greatly simplified, and only two sets of sector gears and rack transmission mechanisms are used to complete the required movement of the push needle linkage assembly and the retracting linkage assembly, and the moving distance of the push needle linkage assembly and the retracting linkage assembly is more accurate, and can be accurately calculated by the length of the meshing part of the sector gear and the rack, thereby further improving the accuracy of the operation.
3) The ocular implant delivery device can complete the surgical operation by unidirectionally rotating the rotating wheel, which is easy to operate, and the friction between the rotating wheel and the outer shell is very small. The force of rotating the rotating wheel can be transmitted to the gear rack mechanism, reducing the loss of pushing force, making the surgical operation more labor-saving, and the system stability and reliability are higher.

### BRIEF DESCRIPTION OF THE DRAWINGS

FIG. 1 is a structural schematic view of an ocular implant delivery device according to the Embodiment 1 of the present application;
FIG. 2 is a schematic structural view of an internal structure of a delivery device according to the Embodiment 1 of the present application;
FIG. 3 is a structural schematic view of a connection structure of a puncture needle seat and a puncture needle according to the Embodiment 1 of the present application;
FIG. 4 is a schematic structural view of a connection structure of a push needle linkage assembly, a retracting linkage assembly, and a rotating wheel according to some embodiments of the present application;
FIG. 5 is a schematic structural view of FIG. 3;
FIG. 6 is a schematic cross-sectional view along A-A.

The reference signs in the drawings are as follows:
1. Housing; 11. Cylinder portion; 12. Gripping portion; 13. Conical portion; 14. Though hole; 15. Opening; 16. Stop block; 17. Rotating wheel mount; 2. Sleeve; 3. Puncture needle; 31. Puncture needle seat; 4: Rotating wheel; 41. Rotating shaft; 42. Sector gear; 43. tooth; 5. Push needle linkage assembly; 51.Push needle; 52. Push needle link; 53. First rack; 6. Retracting linkage assembly; 61. Connector; 62. Retracting link; 63. Second rack; 64. Stop seat; 7. Operating-sliding sleeve; 71. Rack portion; 72. Spring sheet; 8. Implant; 81. Plug.

### DETAILED DESCRIPTION

The technical scheme of the present application is described clearly and completely below through embodiments. Obviously, the described embodiments are only a part of the embodiments of the present application, rather than all the embodiments.

It should be noted that the distal end refers to the end facing away from the doctor during ocular implant operation, and the proximal end refers to the end close to the doctor during ocular implant operation. It can be interpreted that the distal end refers to the end close to the patient's eye that needs operation, and the proximal end is the end facing away from the patient's eye.

### Embodiment 1

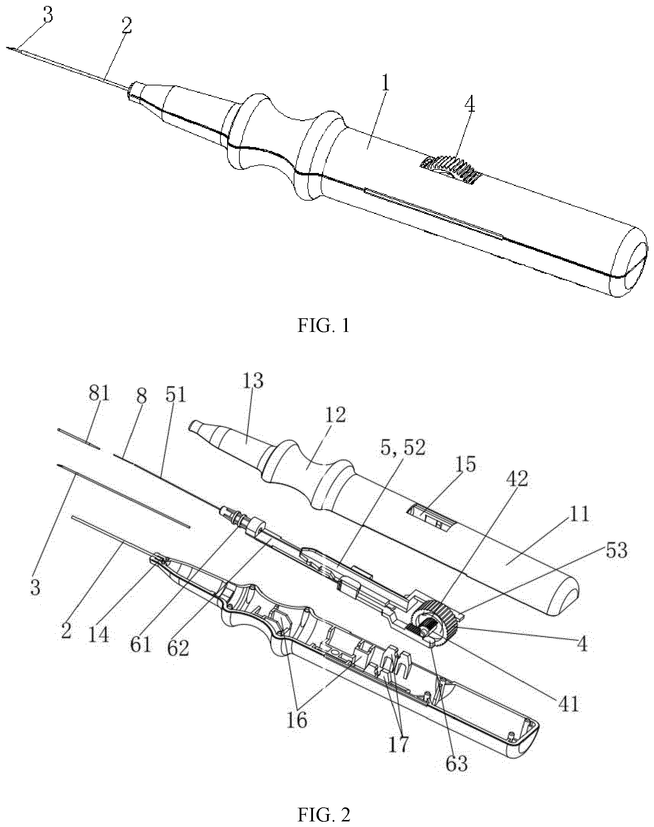

An ocular implant delivery device as shown in FIGS. 1-4 includes a housing 1, a sleeve 2, a puncture needle 3, a rotating wheel 4, a push needle linkage assembly 5, and a retracting linkage assembly 6. The rotating wheel 4 is drivingly connected to the push needle linkage assembly 5 and the retracting linkage assembly 6 via a rack and pinion mechanism, so that the push needle linkage assembly 5 is driven to move toward the distal end of the housing 1 or the retracting linkage assembly 6 is driven to move away from the distal end of the housing 1 by rotating wheel 4 rotating.

The housing 1 is a split molded part, and the structure of the housing 1 can be selected in many ways; for example, as shown in FIG. 2, the housing 1 is divided into an upper housing and a lower housing from the proximal end to the distal end, and the upper housing and the lower housing can be fixedly connected by glue, by buckles, or by bolts. Another solution is that the housing is divided into two parts along the longitudinal direction, and the two parts are connected by threaded connection, buckle connection, glue fixing connection, or the like.

The structure shown in FIG. 1 is taken as an example for explanation below. The housing 1 includes an upper housing and a lower housing, which are fixedly connected. The upper housing and the lower housing each includes a cylinder portion 11, a gripping portion 12 and a conical portion 13 from the proximal end to the distal end. The cylinder portion 11, the gripping portion 12 and the conical portion 13 are fixedly connected or integrally formed. A through hole 14 for connecting the sleeve 2 is disposed on the conical portion 13. With the gripping portion 12, it is convenient for the doctor to hold the ocular implant delivery device during the operation, and the specific shape of the housing 1 can be adjusted and designed according to the convenience of operation.

As shown in FIG. 2, a rotating wheel mount 17 is disposed on the inner wall surface of the cylinder portion of the housing 1, and the rotating wheel 4 is assembled on the rotating wheel mount 17 via a rotating shaft. The rotating wheel mount 17 is provided to facilitate the assembly of the rotating wheel 4 and enable the rotating wheel 4 to rotate flexibly.

The housing 1 is provided with an opening 15 at a position corresponding to the rotating wheel 4, so that the doctor or other surgical operator can operate the surgical procedure by rotating the rotating wheel at the opening 15. Alternatively, the outer periphery of the rotating wheel 4 can be partially protruded from the opening or aligned with the opening of the housing 1.

Inside the housing 1, a stop block 16 is disposed on the inner wall surface of at least one of the cylinder portion 11, the gripping portion 12 and the conical portion 13, and the stop block 16 is used to limit the radial position of the push needle linkage assembly 5 and the retracting linkage assembly 6. With sufficient limit on the housing 1, the retraction link 62 and the push needle link 52 can be prevented from being deviated radially in the housing 1, thereby improving the accuracy of the operation.

As shown in FIG. 2, a sleeve 2 is disposed at the distal end of the housing 1. The sleeve 2 is a hollow tubular object, which is fixedly connected to the distal through hole 14 of the housing 1 by gluing or injection molding or other clamping mechanisms. The sleeve 2 is used to protect the needle tube of the puncture needle 3 and to support the trabecular meshwork tissue when the puncture needle 3 enters the subconjunctival cavity during the operation, thereby limiting the insertion length of the puncture needle.

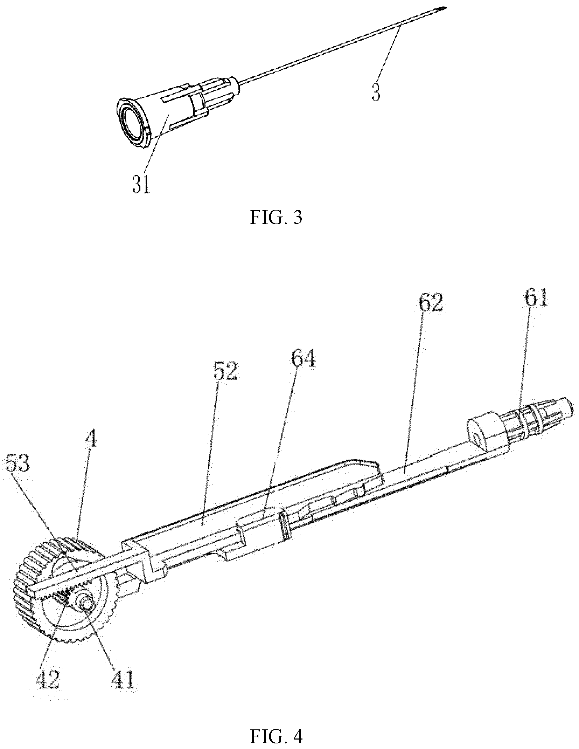

Optionally, a sleeve seat is provided at the proximal end of the sleeve 2, and the sleeve 2 is fixedly connected to the sleeve seat by gluing or injection molding. The sleeve seat is disposed in the housing 1 at a position close to the through hole 14, and the sleeve 2 is fixedly connected to the housing 1, and the connection method can be a fixed connection method such as gluing, injection molding or clamping. The puncture needle 3 is a needle with a hollow cylindrical structure, and its material can be a material specified in the medical field. An oblique opening is provided at the needle tip of the puncture needle 3. The puncture needle 3 passes through the hole of the sleeve 2, and has a clearance fit with the sleeve 2 to enable the puncture needle 3 to pass freely in the sleeve 2. The puncture needle 3 can be directly fixedly connected to the distal end of the retracting linkage assembly 6, or the puncture needle 3 can be fixedly connected to the puncture needle seat 31 by gluing or injection molding, or other clamping mechanisms. As shown in FIG. 3, the puncture needle seat 31 is fixedly connected to the retracting linkage assembly 6.

The puncture needle 3 can pass through the hollow hole of the sleeve 2 and extend to the outer side of the distal end of the sleeve 2. The oblique opening of the puncture needle 3 completely exposes a distance of the sleeve 2, thereby controlling the depth of the puncture needle 3 penetrating into the conjunctiva of the eye. The puncture needle 3 and the puncture needle seat 31 are retained in the internal space composed of the upper housing and lower housing, and it can retract only, but cannot move toward the distal end.

The implant 8 can be a tubular object made of a flexible material, and the tubular inner cavity of the implant 8 forms an aqueous humor outlet channel. The implant 8 has a certain arch along the axial direction. Before the operation, the implant 8 is placed in the inner cavity of the puncture needle 3 and is retained by the distal end of the push needle. A certain friction force is generated between the outer wall of the implant 8 and the inner wall of the puncture needle 3, which can stabilize the position of the implant 8 in the hole of the puncture needle 3.

As shown in FIG. 2 and FIG. 4, two ends of the rotating shaft 41 extend to two sides of the rotating wheel, respectively. Sector gears 42 are respectively disposed on the rotating shafts on two sides of the rotating wheel 4, and the central angle of the sector gear 42 is in a range of 30° to 180°. For example, in the schemes shown in FIG. 2 and FIG. 4, the central angle of the sector gear 42 is from 30° to 60°. With this arrangement, both the push needle linkage assembly 5 and the retracting linkage assembly 6 can have sufficient moving distance to ensure the smooth completion of the ocular implantation operation, and only one of the push needle linkage assembly 5 and the retracting linkage assembly 6 can be in a moving state to avoid the push needle linkage assembly 5 and the retracting linkage assembly 6 moving at the same time to affect the operation.

As shown in FIG. 2 and FIG. 4, the push needle linkage assembly 5 includes a push needle 51, a push needle link 52, and a first rack 53. The proximal end of the push needle 51 is fixedly connected to the distal end of the push needle link 52, and the distal end of the push needle link is fixedly connected to the first rack 53. The first rack 53 is horizontally arranged, and the teeth on the first rack 53 are disposed toward the rotating shaft 41.

The push needle linkage assembly 5 further includes a folded corner portion which is disposed at an end of the push needle link 52 close to the first rack 53 and fixedly connected to the first rack 53. The folded corner portion has an extending direction that deviates from an extending direction of the first rack 53, and extends from the first rack 53 in a direction toward the retracting linkage assembly 6. With setting the folded corner portion, the distance between the push needle link and the retracting linkage assembly is reduced, the first rack can be arranged horizontally and the teeth on the first rack can be disposed toward the rotating shaft, and the push needle link can be arranged at the distal end of the rotating wheel, thereby making the internal structure of the ocular implant delivery device more compact.

As shown in FIG. 2 and FIG. 4, the retracting linkage assembly 6 includes a connector 61, a retracting link 62, and a second rack 63. The connector 61 is fixedly connected to the distal end of the retracting link 62. The connector 61 is configured to connect the puncture needle 3. The second rack 63 is fixedly connected to the proximal end of the retracting link 62. The second rack 63 is horizontally arranged, and the teeth on the second rack 63 are disposed toward the rotating shaft 41. In order to reduce the distance between the push needle link 52 and the retracting link 62 to enable the internal structures of the ocular implant delivery device more compact, a folded corner portion is provided at the connection portion between the retracting link 62 and the second rack 63. The extending direction of the folded corner portion deviates from the second rack and extends from the second rack toward the direction close to the push needle linkage assembly. With the folded corner portion, the second rack can be arranged horizontally and the teeth on the first rack can be arranged toward the rotating shaft, and the retracting link can be arranged at the distal end of the rotating wheel, thereby making the internal structures of the ocular implant delivery device more compact.

As a first implementation scheme, the rack 53 on the push needle linkage assembly 5 is disposed above the rotating shaft 41 of the rotating wheel, the second rack 63 on the retracting linkage assembly 6 is disposed below the rotating shaft 41 of the rotating wheel. The sector gear 42 on the rotating shaft 41 of the rotating wheel corresponding to the first rack 53 intermeshes with the first rack 53 on the push needle linkage assembly 5 in the initial state, that is, in the initial state, the sector gear 42 on the rotating shaft 41 of the rotating wheel corresponding to the first rack 53 is disposed at the upper part of the rotating shaft 41, and the sector gear 42 on the rotating shaft 41 of the rotating wheel corresponding to the second rack 63 is also disposed at the upper part of the rotating shaft 41 in the initial state .

It should be noted that the above and below here are relative concepts, which are relative concepts between the rack and the rotating shaft, indicating that for the operator, the rack of the push needle linkage assembly and the rack of the retracting linkage assembly are respectively at the top and the bottom relative to the rotating shaft.

In the initial state, the sector gear 42 on the rotating shaft 41 of the rotating wheel corresponding to the first rack 53 is disposed at the upper part of the rotating shaft 41. At this time, the first rack 53 and the corresponding sector gear 42 are in the initial contact intermeshing state, so that when the rotating wheel rotates, the corresponding sector gear 42 rolls along the first rack 53 until it is disengaged from the first rack 53, and the push needle linkage assembly completes the movement toward the distal direction. In the initial state, the sector gear 42 on the rotating shaft 41 of the rotating wheel corresponding to the second rack 63 is also disposed at the upper part of the rotating shaft 41, that is, the second rack 63 and the corresponding sector gear 42 are in a disengaged state, and in the process of the push needle linkage assembly moving toward the distal direction, a disengaged state is maintained until the push needle linkage assembly completes the movement toward the distal direction, the second rack 62 and the corresponding sector gear 42 being just in the initial contact intermeshing state. When the rotating wheel continues to rotate, the corresponding sector gear 42 rolls along the second rack 6, thereby realizing that the retracting linkage assembly moves toward the proximal direction. Therefore, both the push needle linkage assembly 5 and the retracting linkage assembly 6 can realize the advancement and retraction in sequence.

It can be understood that in other application scenarios, in the initial state, the first rack 53 and the corresponding sector gear 42 may not be in the initial contact intermeshing, but are in the initial contact intermeshing after the rotating wheel rotates a certain angle (the push needle linkage assembly is in a stationary state when the rotation angle is reached). Similarly, the second rack 63 and the corresponding sector gear 42 may not be in the initial contact intermeshing after the push needle linkage assembly 5 has just completed moving toward the distal direction, but are in the initial contact intermeshing after the rotating wheel 4 continues to rotate a certain angle (the retracting linkage assembly is in a stationary state when the rotation angle is reached).

With this arrangement, when the rotating wheel rotates toward the distal direction of the housing 1, the push needle linkage assembly 5 can drive the push needle linkage assembly 5 to move toward the distal direction of the housing 1, and the retracting linkage assembly 6 remains stationary. When the push needle linkage assembly 5 completes the delivery of the ocular implant 8, the rotating wheel continues to be rotated, the push needle linkage assembly 5 no longer moves, and the retracting linkage assembly 6 moves toward the proximal direction, thereby realizing the linear contraction of the puncture needle 3 and improving the safety of the operation.

The ocular implant delivery device in this embodiment can complete the delivery operation of the ocular implant 8 and the retracting operation of the puncture needle 3 by rotating the rotating wheel 4 when performing an ocular implant delivery operation, thereby completing the ocular implant operation.

The specific process is that in the initial state, the side of the rotating wheel 4 corresponding to the push needle linkage assembly 5 and the sector gear 42 on the rotating shaft 41 are located at the upper part of the rotating shaft 4. The sector gear 42 intermeshes with the first rack 53, the sector gear 42 on the rotating shaft 41 on the other side of the rotating wheel 4 is also located at the upper part of the rotating shaft 4, and the second rack 63 at the proximal end of the retracting link 62 is located at the lower part of the rotating shaft 4, so the second rack 63 does not intermesh with the sector gear 42 on the rotating shaft 4.

The implant 8 is pre-installed in the inner hole of the puncture needle 3, and abuts against the push needle 51 of the push needle linkage assembly 5. The distal end of the implant 8 is restrained by a plug 81 to prevent the implant 8 from falling. Before implantation, the plug 81 is pulled out to unlock the ocular implant delivery device, and then the puncture needle 3 is used to puncture the surgical site of the patient. Because the length of the puncture needle 3 exposing out of the sleeve 2 is fixed, the sleeve 2 can limit the puncture depth of the puncture needle 3. After the puncture is completed, the implantation operation begins.

In the first stage, as shown in the direction of the arrow in FIG. 2, the rotating wheel 4 is rotated toward the distal end. As shown in the direction of the arrow in FIG. 4, the sector gear 42 on the rotating shaft 41 intermeshes with the first rack 53 at the proximal end of the push needle linkage assembly 5, thereby driving the push needle linkage assembly 5 to move toward the distal end of the housing 1, and pushing the implant 8 through the puncture hole to reach the patient's intraocular subconjunctival cavity to complete the delivery operation of the ocular implant 8. At the same time, as shown in the direction of the arrow in FIG. 2, the sector gear 42 on the rotating shaft 41 on the other side of the rotating wheel 4 does not intermesh with the second rack 63 on the retracting linkage assembly 6, so the retracting linkage assembly 6 does not move in the axial direction.

When the rotating wheel 4 continues to be rotated toward the distal direction to enter the second stage, the first rack 53 is separated from the corresponding sector gear 42, so that the rotating wheel 4 no longer drives the needle linkage assembly 5 to move, and the second rack 63 gradually engages with the sector gear 42 on the corresponding side. Because the second rack 63 is located below the sector gear 42, when the rotating wheel continues to rotate, it can drive the retracting linkage assembly 6 to move toward the proximal direction. With a stop block 16 disposed in the housing 1, the radial movement of the retracting link 62 can be limited, so that it can only move along the axial direction of the housing 1 toward the proximal direction, driving the puncture needle 3 to move into the sleeve 2 to complete the needle retracting operation.

At this point, the delivery operation of the ocular implant is completed. For doctors and other operators, they only need to operate the rotating wheel 4 to complete the implant delivery process, which is simple and easy.

### Embodiment 2

On the basis of Embodiment 1, the differences from Embodiment 1 are that the first rack 53 is disposed below the rotating shaft 41 of the rotating wheel, the second rack 63 is disposed above the rotating shaft 41 of the rotating wheel, and the sector gear 42 on the rotating shaft 41 of the rotating wheel corresponding to the first rack 53 intermeshes with the first rack 53 on the push needle linkage assembly 5 in the initial state, that is, the sector gear 42 on the rotating shaft 41 of the rotating wheel corresponding to the first rack 53 is located at the lower part of the rotating shaft 41 in the initial state, and the sector gear 42 on the rotating shaft 41 of the rotating wheel corresponding to the second rack 63 is located at the lower part of the rotating shaft 41 in the initial state. The principle of this scheme is exactly the same as that of the above-mentioned Embodiment 1, except that when performing the delivery operation of the ocular implant, the rotating wheel 4 rotates toward the proximal direction of the housing 1.

The structure and working principle of this embodiment are basically the same as those of Embodiment 1, except that the first rack 53 and the second rack 63 are arranged at positions opposite to those of Embodiment 1, and during the operation, the initial position and the rotation direction of the rotating wheel 4 are opposite to those of Embodiment 1. Although there are no drawings to illustrate this embodiment, it does not affect the understanding of the technical personnel of this embodiment.

The ocular implant delivery device in this embodiment can complete the delivery operation of the ocular implant 8 and the retracting operation of the puncture needle 3 by rotating the rotating wheel 4 when performing an ocular implant delivery operation, thereby completing the ocular implant operation.

The specific process is that in the initial state, the sector gear 42 on the rotating shaft 41 at the side of the rotating wheel 4 corresponding to the push needle linkage assembly 5 is located at the lower part of the rotating shaft 41 and intermeshes with the first rack 53. The sector gear 42 on the rotating shaft 41 at the other side of the rotating wheel 4 is also located at the lower part of the rotating shaft 41, and the second rack 63 at the proximal end of the retracting link 62 is located at the upper part of the rotating shaft 41, so the second rack 63 does not intermesh with the sector gear 42 on the rotating shaft 41.

The implant 8 is pre-installed in the inner hole of the puncture needle 3, and abuts against the push needle 51 of the push needle linkage assembly 5, and the distal end of the implant 8 is limited by a plug 81 to prevent the implant 8 from falling. Before implantation, the plug 81 is pulled out to unlock the ocular implant delivery device, and then the puncture needle 3 is used to puncture the surgical site of the patient. Because the length of the puncture needle 3 exposing out of the sleeve 2 is fixed, the sleeve 2 can limit the puncture depth of the puncture needle 3. After the puncture is completed, the implantation operation begins.

In the first stage, referring to FIG. 2, the rotating wheel 4 is rotated toward the proximal end of the housing in the opposite direction to the direction of the arrow in FIG. 2, and the sector gear 42 on the rotating shaft 41 intermeshes with the first rack 53 at the proximal end of the push needle linkage assembly 5, thereby driving the push needle linkage assembly 5 to move toward the distal end of the housing 1, pushing the implant 8 through the puncture hole to reach the patient's intraocular subconjunctival cavity to complete the delivery operation of the ocular implant 8; at the same time, the sector gear 42 on the rotating shaft 41 on the other side of the rotating wheel does not intermesh with the second rack 63 on the retracting linkage assembly 6, so that the retracting linkage assembly 6 will not move in the axial direction.

When the rotating wheel 4 continues to rotate toward the proximal direction to enter the second stage, the first rack 53 is separated from the corresponding sector d gear 42, so that the rotating wheel 4 no longer drives the push needle linkage assembly 5 to move, and the second rack 63 gradually engages with the sector gear 42 on the corresponding side. Because the second rack 63 is located above the sector gear 42, when the rotating wheel 4 continues to be rotated, it can drive the retracting linkage assembly 6 to move toward the proximal direction. Because a stop block 16 is disposed in the housing 1, the radial movement of the retracting link 62 can be limited, so that it can only move along the axial direction of the housing 1 toward the proximal direction, driving the puncture needle 3 to move into the sleeve 2 to complete the needle retracting operation.

At this point, the delivery operation of the ocular implant is completed. For doctors and other operators, they only need to operate the rotating wheel 4 to complete the implant delivery process, which is simple and easy.

### Embodiment 3

Optionally, as shown in FIG. 2 and FIG. 4, the connector 61 of the retracting link may further be provided with a through hole along the extending direction of the retracting link, and the push needle linkage assembly passes through the through hole and extends into the inner hole of the puncture needle. With the through hole, the push needle of the push needle linkage assembly is guided, thereby improving the safety of the operation. Optionally, along the extending direction of the retracting link, the inner diameter of the through hole is smaller than the maximum dimension of the push needle link of the push needle linkage assembly along the radial direction of the through hole. In this way, the moving distance of the push needle linkage assembly is limited, thereby preventing the push needle linkage assembly from moving a distance exceeding the expected range to improve the safety of the operation.

Optionally, the ocular implant delivery device may further include a stop seat 64 disposed on the retracting link, and the push needle link 52 passes through the stop seat 64, to enable the two to form an integral structure. It can be understood that in other application scenarios, a Stop seat may also be provided on the push needle link 52, so that the retracting link passes through the Stop seat on the push needle link and forms an integral structure with the push needle link. The design of this integral structure makes the space utilization rate high and the internal structure more compact. Optionally, the stop seat 64 may include a first extending section and a second extending section intersecting each other, the first extending section is fixedly connected to the second extending section and the retracting link 62 or the push needle link 52, and extends from the retracting link 62 to the push needle link 52 by a predetermined distance. The second extending section extends from the first extending section to a direction facing away from the first extending section, and the orthographic projection of the second extending section falls within the range of the retracting link 62 and the push needle link 52 along the extending direction of the first extending section. With this arrangement, the push needle link 52 or the retracting link 62 passes through the stop seat, so that the distance between the push needle link and the retracting link is limited to form an integral structure.

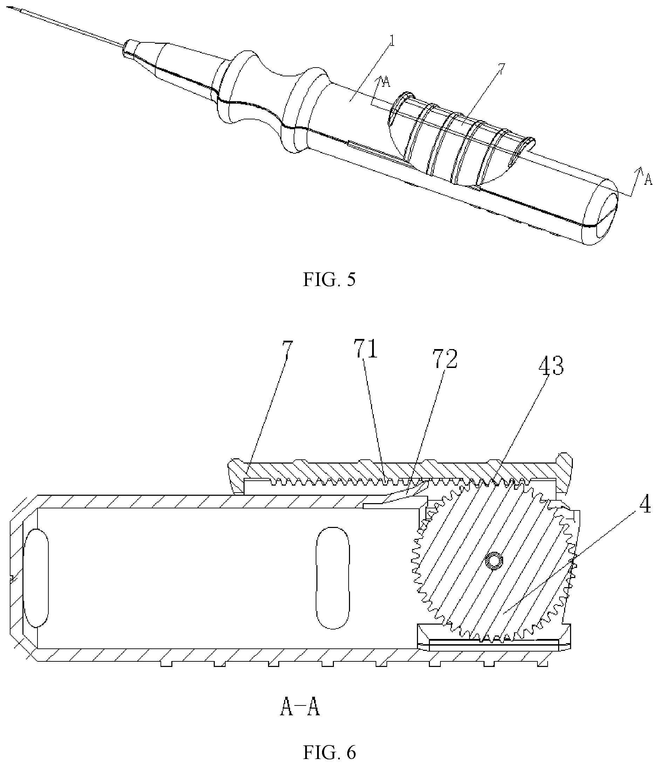

As shown in FIG. 5 and FIG. 6, on the basis of Embodiment 1 or 2, the teeth 43 are provided on the outer peripheral surface of the rotating wheel, an operating-sliding sleeve 7 is provided at a position corresponding to the opening on the housing and has a slip fit with the housing 1, and a rack portion 71 intermeshing with the teeth 43 on the rotating wheel 4 is provided on the inner wall of the operating-sliding 7. With the operating-sliding sleeve 7 provided, it is convenient for the doctor to push the operating-sliding sleeve by hand to perform the operation. Compared with the solution of directly rotating the rotating wheel to perform the operation, the flexibility and convenience of the operation of the ocular implant delivery device can be greatly improved, making the operation process more labor-saving and more efficient.

Optionally, as shown in FIG. 6, a spring sheet 72 is provided on the outer wall of the housing 1, extends to the outside of the housing 1, and cooperates with the rack portion 71 on the inner wall of the operating-sliding sleeve 7. By providing the spring sheet 72, when the doctor pushes the operating-sliding sleeve 7 to perform operation, there will be obvious tactile feedback, and each time the rotating wheel 4 moves a tooth, and the spring sheet 72 will engage with the tooth 43, which can effectively prevent the reverse rotation of the rotating wheel due to misoperation during operation to improve the safety of the operation of the ocular implant delivery device. And by providing the spring sheet, it can be prevented that the doctor uses too much force during operation and the rotating wheel rotates too fast to affect the quality of the operation, further improving the safety of the operation.

When the ocular implant delivery device in the present embodiment is used, the implant 8 that meets the surgical conditions is first loaded into the sterile puncture needle 3, and then according to surgical conditions, the eyes of the patient are treated correspondingly, such as anesthesia, sterilization, or the like. When the operation is to be performed, the puncture needle 3 of the ocular implant delivery device is used to puncture the part of the patient where the ocular implant needs to be implanted. The puncture depth of the puncture needle 3 can be limited by the sleeve 2 to improve the accuracy of the operation. After the puncture is in place, the doctor holds the holding portion of the ocular implant delivery device and slowly pushes the operating-sliding sleeve 7 toward the distal end or proximal end of the housing 1. The operating-sliding sleeve can drive the rotating wheel to rotate, and then can first drive the push needle linkage assembly to move toward the distal end of the housing 1. Because a stop block 16 for limiting the push needle link 52 is provided in the housing 1, the radial displacement of the push needle link 52 can be avoided, so that it can only move linearly toward the distal end of the housing 1, driving the push needle 51 to move toward the puncture needle 3, and pushing the ocular implant through the puncture hole 3 to reach the patient's intraocular subconjunctival cavity. Continuing to push the operating-sliding sleeve 7 in the same direction, the retracting link 62 will move toward the proximal end of the housing. During the movement, because the retracting link 62 is affected by the stop block 16 in the housing 1, it can only move linearly from the distal end to the proximal end of the housing 1, driving the puncture needle 3 to retract into the sleeve 2, and completing the needle retracting operation. The implant 8 is delivered to a specific position in the eye by using the ocular implant delivery device, and the tubular inner cavity of the implant 8 forms an aqueous humor outflow channel to discharge the aqueous humor in the eye, thereby achieving the purpose of reducing intraocular pressure.

The ocular implant delivery device of the present application has a compact structure, ingenious design, and is easy to operate. It can greatly improve the accuracy, safety, and efficiency of operation, reduce the difficulty and risk of surgical operations, alleviate the pain of patients, and improve the success rate of operation.

Although the embodiments of the present application have been shown and described, it will be appreciated by those skilled in the art that various changes, modifications, substitutions and variations may be made to the embodiments without departing from the principles of the present application, and that the scope of the present application is defined by the appended claims.

## Claims

1. An ocular implant delivery device, comprising: a housing (1), a rotating wheel (4), a push needle linkage assembly (5), a retracting linkage assembly (6), a sleeve (2), and a puncture needle (3),
wherein the housing (1) is provided with a through hole at a distal end of the housing (1), and the sleeve (2) extends from an inside of the housing (1) to an outside of the housing (1) through the through hole and is fixedly connected to the housing (1);
wherein the puncture needle (3) runs through the sleeve (2), a proximal end of the puncture needle (3) is fixedly connected to the retracting linkage assembly (6), and a push needle (51) of the push needle linkage assembly (5) runs through an inner hole of the puncture needle (3);
wherein the rotating wheel (4) is connected to an inside of the housing (1) via rotating shafts (41), an opening (15) corresponding to the rotating wheel (4) is provided on the housing (1), and the rotating wheel (4) is drivingly connected to the push needle linkage assembly (5) and the retracting linkage assembly (6) via a rack and pinion mechanism, so that the push needle linkage assembly (5) is driven to move toward the distal end of the housing (1) or the retracting linkage assembly (6) is driven to move away from the distal end of the housing (1) by the rotating wheel (4) rotating;
and **characterized in that**:
the rotating shafts (41) disposed on two sides of the rotating wheel (4) each are provided with a sector gear (42), and a central angle of the sector gear (42) is in a range of 30° to 180°; and
the push needle linkage assembly (5) and the retracting linkage assembly (6) each are provided with a rack at a proximal end, the racks on the push needle linkage assembly (5) and on the retracting linkage assembly (6) extending to the two sides of the rotating wheel (4) respectively, and each of the racks being configured to intermesh with the sector gear (42) on the rotating shaft (41) on the corresponding side of the rotating wheel (4).

2. The ocular implant delivery device according to claim 1, wherein the sector gears (42) on the rotating shafts (41) on the two sides of the rotating wheel (4) are disposed on the same side of the rotating shafts (41) along a radial direction of the rotating shaft (41), and the racks disposed on the push needle linkage assembly (5) and on the retracting linkage assembly (6) are arranged at intervals along a radial direction of the rotating wheel (4), and each of the racks is configured to intermesh with the sector gear (42) on the rotating shaft (41) on the corresponding side.

3. The ocular implant delivery device according to claim 2, wherein the first rack (53) on the push needle linkage assembly (5) is disposed above the rotating shafts (41) of the rotating wheel (4), and the second rack (63) on the retracting linkage assembly (6) is disposed below the rotating shafts (41) of the rotating wheel (4); and
the sector gear (42) on the rotating shaft (41) of the rotating wheel (4) corresponding to the first rack (53) on the push needle linkage assembly (5) intermeshes with the first rack (53) on the push needle linkage assembly (5) in an initial state, and the sector gear (42) on the rotating shaft (41) of the rotating wheel (4) corresponding to the second rack (63) on the retracting linkage assembly (6) is located on the upper part of the rotating shaft (41) in the initial state.

4. The ocular implant delivery device according to claim 2, wherein the first rack on the push needle linkage assembly (5) is disposed below the rotating shafts (41) of the rotating wheel (4), and the second rack (63) on the retracting linkage assembly (6) is disposed above the rotating shafts (41) of the rotating wheel (4); and
the sector gear (42) on the rotating shaft (41) of the rotating wheel (4) corresponding to the first rack (53) on the push needle linkage assembly (5) intermeshes with the first rack (53) on the push needle linkage assembly (5) in an initial state, and the sector gear (42) on the rotating shaft (41) of the rotating wheel (4) corresponding to the second rack (63) on the retracting linkage assembly (6) is located on the lower part of the rotating shaft (41) in the initial state.

5. The ocular implant delivery device according to claim 1, wherein the push needle linkage assembly (5) comprises the push needle (51), a push needle link (52), and the first rack (53); and
a proximal end of the push needle (51) is fixedly connected to a distal end of the push needle link (52), a proximal end of the push needle link (52) is fixedly connected to the first rack (53) horizontally arranged, and teeth (43) on the first rack (53) are disposed toward the rotating shaft (41).

6. The ocular implant delivery device according to claim 1, wherein the retracting linkage assembly (6) comprises a connector (61), a retracting link (62), and the second rack (63) horizontally arranged, the connector (61) being fixedly connected to a distal end of the retracting link (62), the second rack (63) being fixedly connected to the proximal end of the retracting link (62), and teeth (43) on the second rack (63) being disposed toward the rotating shaft (41).

7. The ocular implant delivery device according to claim 1, further comprising a stop seat (64) configured to define a distance between the push needle linkage assembly (5) and the retracting linkage assembly (6).

8. The ocular implant delivery device according to claim 7, wherein the stop seat (64) comprises a first extending section and a second extending section intersecting each other;
the first extending section is fixedly connected to the second extending section and is fixedly connected to the retracting linkage assembly (6) or the push needle linkage assembly (5), and extends from the retracting linkage assembly (6) to the push needle linkage assembly (5) by a predetermined distance; and
the second extending section extends from the first extending section in a direction facing away from the first extending section, and an orthographic projection of the second extending section falls within a range of the retracting linkage assembly (6) and the push needle linkage assembly (5) along an extending direction of the first extending section.

9. The ocular implant delivery device according to claim 1, wherein a proximal end of the sleeve (2) is fixedly connected to a sleeve seat which is disposed in the housing (1) at a position close to the through hole, and the sleeve (2) extends through the through hole on the housing (1) to the outside of the housing (1).

10. The ocular implant delivery device according to claim 1, wherein the puncture needle (3) is provided with a puncture needle seat (31) at a proximal end, the puncture needle (3) being fixedly connected to the puncture needle seat (31) and running through the sleeve (2), and the puncture needle seat (31) being fixedly connected to a distal end of the retracting linkage assembly (6).

11. The ocular implant delivery device according to claim 1, wherein the housing (1) comprises an upper housing and a lower housing fixedly connected to the upper housing, the upper housing and the lower housing each comprise a cylinder portion (11), a gripping portion (12), and a conical portion (13) that are fixedly connected together;
a rotating wheel mount (17) is disposed on an inner wall surface of the cylinder portion (11) of the housing (1), and the rotating wheel (4) is fitted on the rotating wheel mount (17) via the rotating shafts (41); and
a stop block (16) is provided on an inner wall surface of at least one of the cylinder portion (11), the gripping portion (12), and the conical portion (13), and the stop block (16) is configured to limit the push needle linkage assembly (5) and the retracting linkage assembly (6) in a radial direction.

12. The ocular implant delivery device according to claim 1, wherein the rotating wheel (4) is provided with teeth (43) on an outer circumferential surface, and the housing (1) is provided with an operating-sliding sleeve (7) at a position corresponding to the opening (15), the operating-sliding sleeve (7) being slidably fitted with the housing (1); and
the operating-sliding sleeve (7) is provided with a rack portion (71) on an inner wall, the rack portion (71) intermeshing with the teeth (43) on the rotating wheel (4).

13. The ocular implant delivery device according to claim 12, wherein the housing (1) is provided with a spring sheet (72) on an outer wall, the spring sheet (72) extending to the outside of the housing (1) and cooperating with the rack portion (71) on an inner wall of the operating-sliding sleeve (7).

## Patentansprüche

1. Eine Vorrichtung zum Abgeben eines okularen Implantats, umfassend: ein Gehäuse (1), ein Drehrad (4), eine Vorschubnadel-Gelenkanordnung (5), eine Rückzugs-Gelenkanordnung (6), eine Hülse (2) und eine Punktionsnadel (3),
wobei das Gehäuse (1) an seinem distalen Ende mit einer Durchgangsbohrung versehen ist, und sich die Hülse (2) von einem Inneren des Gehäuses (1) durch die Durchgangsbohrung nach außen zum Gehäuse (1) erstreckt und fest mit dem Gehäuse (1) verbunden ist;
wobei die Punktionsnadel (3) die Hülse (2) durchläuft, ein proximales Ende der Punktionsnadel (3) fest mit der Rückzugs-Gelenkanordnung (6) verbunden ist, und eine Vorschubnadel (51) der Vorschubnadel-Gelenkanordnung (5) durch ein Innenloch der Punktionsnadel (3) verläuft;
wobei das Drehrad (4) über Drehachsen (41) mit einem Inneren des Gehäuses (1) verbunden ist, an dem Gehäuse (1) eine dem Drehrad (4) entsprechende Öffnung (15) vorgesehen ist, und das Drehrad (4) über ein Zahnstangen- und Ritzelgetriebe antriebsmäßig mit der Vorschubnadel-Gelenkanordnung (5) und der Rückzugs-Gelenkanordnung (6) verbunden ist, so dass die Vorschubnadel-Gelenkanordnung (5) durch Drehung des Drehrades (4) angetrieben wird, sich zum distalen Ende des Gehäuses (1) hin zu bewegen, oder die Rückzugs-Gelenkanordnung (6) angetrieben wird, sich vom distalen Ende des Gehäuses (1) weg zu bewegen;
und **dadurch gekennzeichnet, dass**:
die an zwei Seiten des Drehrades (4) angeordneten Drehachsen (41) jeweils mit einem Sektorzahnrad (42) versehen sind, und ein Zentriwinkel des Sektorzahnrades (42) in einem Bereich von 30° bis 180° liegt; und
die Vorschubnadel-Gelenkanordnung (5) und die Rückzugs-Gelenkanordnung (6) jeweils an ihrem proximalen Ende mit einer Zahnstange versehen sind, wobei sich die Zahnstangen an der Vorschubnadel-Gelenkanordnung (5) und an der Rückzugs-Gelenkanordnung (6) jeweils zu den zwei Seiten des Drehrades (4) erstrecken, und jede der Zahnstangen konfiguriert ist, mit dem Sektorzahnrad (42) auf der Drehachse (41) auf der entsprechenden Seite des Drehrades (4) in Eingriff zu kommen.

2. Die Vorrichtung zum Abgeben eines okularen Implantats nach Anspruch 1, **dadurch gekennzeichnet, dass** die Sektorzahnräder (42) an den Drehachsen (41) auf den zwei Seiten des Drehrades (4) in radialer Richtung der Drehachse (41) auf derselben Seite der Drehachsen (41) angeordnet sind, und die an der Vorschubnadel-Gelenkanordnung (5) und an der Rückzugs-Gelenkanordnung (6) angeordneten Zahnstangen in radialer Richtung des Drehrades (4) beabstandet angeordnet sind, und jede der Zahnstangen konfiguriert ist, mit dem Sektorzahnrad (42) auf der Drehachse (41) auf der entsprechenden Seite in Eingriff zu kommen.

3. Die Vorrichtung zum Abgeben eines okularen Implantats nach Anspruch 2, **dadurch gekennzeichnet, dass** die erste Zahnstange (53) an der Vorschubnadel-Gelenkanordnung (5) oberhalb der Drehachsen (41) des Drehrades (4) angeordnet ist, und die zweite Zahnstange (63) an der Rückzugs-Gelenkanordnung (6) unterhalb der Drehachsen (41) des Drehrades (4) angeordnet ist; und
in einem Anfangszustand das Sektorzahnrad (42) an der Drehachse (41) des Drehrades (4), das der ersten Zahnstange (53) an der Vorschubnadel-Gelenkanordnung (5) entspricht, mit der ersten Zahnstange (53) an der Vorschubnadel-Gelenkanordnung (5) in Eingriff ist, und das Sektorzahnrad (42) an der Drehachse (41) des Drehrades (4), das der zweiten Zahnstange (63) an der Rückzugs-Gelenkanordnung (6) entspricht, sich im Anfangszustand im oberen Teil der Drehachse (41) befindet.

4. Die Vorrichtung zum Abgeben eines okularen Implantats nach Anspruch 2, **dadurch gekennzeichnet, dass** die erste Zahnstange an der Vorschubnadel-Gelenkanordnung (5) unterhalb der Drehachsen (41) des Drehrades (4) angeordnet ist, und die zweite Zahnstange (63) an der Rückzugs-Gelenkanordnung (6) oberhalb der Drehachsen (41) des Drehrades (4) angeordnet ist; und
in einem Anfangszustand das Sektorzahnrad (42) an der Drehachse (41) des Drehrades (4), das der ersten Zahnstange (53) an der Vorschubnadel-Gelenkanordnung (5) entspricht, mit der ersten Zahnstange (53) an der Vorschubnadel-Gelenkanordnung (5) in Eingriff ist, und das Sektorzahnrad (42) an der Drehachse (41) des Drehrades (4), das der zweiten Zahnstange (63) an der Rückzugs-Gelenkanordnung (6) entspricht, sich im Anfangszustand im unteren Teil der Drehachse (41) befindet.

5. Die Vorrichtung zum Abgeben eines okularen Implantats nach Anspruch 1, **dadurch gekennzeichnet, dass** die Vorschubnadel-Gelenkanordnung (5) die Vorschubnadel (51), ein Vorschubnadel-Gelenk (52) und die erste Zahnstange (53) umfasst; und
ein proximales Ende der Vorschubnadel (51) fest mit einem distalen Ende des Vorschubnadel-Gelenks (52) verbunden ist, ein proximales Ende des Vorschubnadel-Gelenks (52) fest mit der horizontal angeordneten ersten Zahnstange (53) verbunden ist, und Zähne (43) an der ersten Zahnstange (53) zur Drehachse (41) hin ausgerichtet sind.

6. Die Vorrichtung zum Abgeben eines okularen Implantats nach Anspruch 1, **dadurch gekennzeichnet, dass** die Rückzugs-Gelenkanordnung (6) einen Verbinder (61), ein Rückzugs-Gelenk (62) und eine horizontal angeordnete zweite Zahnstange (63) umfasst, wobei der Verbinder (61) fest mit einem distalen Ende des Rückzugs-Gelenks (62) verbunden ist, die zweite Zahnstange (63) fest mit dem proximalen Ende des Rückzugs-Gelenks (62) verbunden ist, und Zähne (43) an der zweiten Zahnstange (63) zur Drehachse (41) hin ausgerichtet sind.

7. Die Vorrichtung zum Abgeben eines okularen Implantats nach Anspruch 1, ferner umfassend einen Anschlagsitz (64), der konfiguriert ist, um einen Abstand zwischen der Vorschubnadel-Gelenkanordnung (5) und der Rückzugs-Gelenkanordnung (6) zu definieren.

8. Die Vorrichtung zum Abgeben eines okularen Implantats nach Anspruch 7, **dadurch gekennzeichnet, dass** der Anschlagsitz (64) einen ersten erstreckten Abschnitt und einen zweiten erstreckten Abschnitt umfasst, die sich gegenseitig kreuzen;
der erste erstreckte Abschnitt fest mit dem zweiten erstreckten Abschnitt verbunden ist und fest mit der Rückzugs-Gelenkanordnung (6) oder der Vorschubnadel-Gelenkanordnung (5) verbunden ist, und sich um eine vorbestimmte Strecke von der Rückzugs-Gelenkanordnung (6) zur Vorschubnadel-Gelenkanordnung (5) erstreckt; und
der zweite erstreckte Abschnitt sich vom ersten erstreckten Abschnitt in einer Richtung weg vom ersten erstreckten Abschnitt erstreckt, und eine orthogonale Projektion des zweiten erstreckten Abschnitts entlang einer Erstreckungsrichtung des ersten erstreckten Abschnitts in einen Bereich der Rückzugs-Gelenkanordnung (6) und der Vorschubnadel-Gelenkanordnung (5) fällt.

9. Die Vorrichtung zum Abgeben eines okularen Implantats nach Anspruch 1, **dadurch gekennzeichnet, dass** ein proximales Ende der Hülse (2) fest mit einem Hülsensitz verbunden ist, der im Gehäuse (1) an einer Stelle nahe der Durchgangsbohrung angeordnet ist, und sich die Hülse (2) durch die Durchgangsbohrung am Gehäuse (1) nach außen zum Gehäuse (1) erstreckt.

10. Die Vorrichtung zum Abgeben eines okularen Implantats nach Anspruch 1, **dadurch gekennzeichnet, dass** die Punktionsnadel (3) an ihrem proximalen Ende mit einem Punktionsnadel-Sitz (31) versehen ist, wobei die Punktionsnadel (3) fest mit dem Punktionsnadel-Sitz (31) verbunden ist und die Hülse (2) durchläuft, und der Punktionsnadel-Sitz (31) fest mit einem distalen Ende der Rückzugs-Gelenkanordnung (6) verbunden ist.

11. Die Vorrichtung zum Abgeben eines okularen Implantats nach Anspruch 1, **dadurch gekennzeichnet, dass** das Gehäuse (1) ein Obergehäuse und ein mit dem Obergehäuse fest verbundenes Untergehäuse umfasst, wobei das Obergehäuse und das Untergehäuse jeweils einen Zylinderabschnitt (11), einen Griffabschnitt (12) und einen Kegelabschnitt (13) umfassen, die fest miteinander verbunden sind;
an einer Innenwandfläche des Zylinderabschnitts (11) des Gehäuses (1) eine Drehradhalterung (17) angeordnet ist, und das Drehrad (4) über die Drehachsen (41) an der Drehradhalterung (17) angebracht ist; und
an einer Innenwandfläche von mindestens einem des Zylinderabschnitts (11), des Griffabschnitts (12) und des Kegelabschnitts (13) ein Anschlagblock (16) vorgesehen ist, und der Anschlagblock (16) konfiguriert ist, um die Vorschubnadel-Gelenkanordnung (5) und die Rückzugs-Gelenkanordnung (6) in einer radialen Richtung zu begrenzen.

12. Die Vorrichtung zum Abgeben eines okularen Implantats nach Anspruch 1, **dadurch gekennzeichnet, dass** das Drehrad (4) an seiner äußeren Umfangsfläche mit Zähnen (43) versehen ist, und das Gehäuse (1) an einer die Öffnung (15) entsprechenden Stelle mit einer Betriebs-Gleithülse (7) versehen ist, wobei die Betriebs-Gleithülse (7) gleitend mit dem Gehäuse (1) ausgestattet ist; und
die Betriebs-Gleithülse (7) an einer Innenwand mit einem Zahnstangenabschnitt (71) versehen ist, wobei der Zahnstangenabschnitt (71) mit den Zähnen (43) am Drehrad (4) in Eingriff kommt.

13. Die Vorrichtung zum Abgeben eines okularen Implantats nach Anspruch 12, **dadurch gekennzeichnet, dass** das Gehäuse (1) an einer Außenwand mit einem Federblech (72) versehen ist, wobei sich das Federblech (72) nach außen zum Gehäuse (1) erstreckt und mit dem Zahnstangenabschnitt (71) an einer Innenwand der Betriebs-Gleithülse (7) zusammenwirkt.

## Revendications

1. Dispositif de délivrance d'implant oculaire, comprenant : un boîtier (1), une roue rotative (4), un ensemble de liaison d'aiguille-poussoir (5), un ensemble de liaison de rétraction (6), une gaine (2) et une aiguille à ponction (3),
dans lequel le boîtier (1) est pourvu d'un trou traversant à une extrémité distale du boîtier (1), et la gaine (2) s'étend à partir d'un intérieur du boîtier (1) vers un extérieur du boîtier (1) à travers le trou traversant et est reliée de manière fixe au boîtier (1) ;
dans lequel l'aiguille à ponction (3) passe à travers la gaine (2), une extrémité proximale de l'aiguille à ponction (3) est reliée de manière fixe à l'ensemble de liaison de rétraction (6), et une aiguille-poussoir (51) de l'ensemble de liaison d'aiguille-poussoir (5) passe à travers un trou interne de l'aiguille à ponction (3) ;
dans lequel la roue rotative (4) est reliée à un intérieur du boîtier (1) par l'intermédiaire des arbres rotatifs (41), une ouverture (15) correspondant à la roue rotative (4) est prévue sur le boîtier (1), et la roue rotative (4) est reliée par entraînement à l'ensemble de liaison d'aiguille-poussoir (5) et à l'ensemble de liaison de rétraction (6) par l'intermédiaire d'un mécanisme crémaillère-pignon, de sorte que l'ensemble de liaison d'aiguille-poussoir (5) soit entraîné à se déplacer vers l'extrémité distale du boîtier (1) ou que l'ensemble de liaison de rétraction (6) soit entraîné à se déplacer pour s'éloigner de l'extrémité distale du boîtier (1) par la rotation de la roue rotative (4) ;
et **caractérisé en ce que** :
les arbres rotatifs (41) disposés sur deux côtés de la roue rotative (4) sont chacun pourvus d'un engrenage sectoriel (42), et un angle central de l'engrenage sectoriel (42) est compris entre 30° et 180° ; et
l'ensemble de liaison d'aiguille-poussoir (5) et l'ensemble de liaison de rétraction (6) sont chacun pourvus d'une crémaillère à une extrémité proximale, les crémaillères sur l'ensemble de liaison d'aiguille-poussoir (5) et sur l'ensemble de liaison de rétraction (6) s'étendant respectivement vers les deux côtés de la roue rotative (4), et chacune des crémaillères étant configurée pour s'engrener avec l'engrenage sectoriel (42) sur l'arbre rotatif (41) du côté correspondant de la roue rotative (4).

2. Dispositif de délivrance d'implant oculaire selon la revendication 1, dans lequel les engrenages sectoriels (42) sur les arbre rotatifs (41) des deux côtés de la roue rotative (4) sont disposées sur le même côté des arbre rotatifs (41) selon une direction radiale de l'arbre rotatif (41), les crémaillères disposées sur l'ensemble de liaison d'aiguille-poussoir (5) et sur l'ensemble de liaison de rétraction (6) sont disposées à intervalles selon une direction radiale de la roue rotative (4), et chacune des crémaillères est configurée pour s'engrener avec l'engrenage sectoriel (42) sur l'arbre rotatif (41) du côté correspondant.

3. Dispositif de délivrance d'implant oculaire selon la revendication 2, dans lequel la première crémaillère (53) sur l'ensemble de liaison d'aiguille-poussoir (5) est disposée au-dessus des arbres rotatifs (41) de la roue rotative (4), et la seconde crémaillère (63) sur l'ensemble de liaison de rétraction (6) est disposée sous les arbres rotatifs (41) de la roue rotative (4) ; et
l'engrenage sectoriel (42) sur l'arbre rotatif (41) de la roue rotative (4) correspondant à la première crémaillère (53) sur l'ensemble de liaison d'aiguille-poussoir (5) s'engrène avec la première crémaillère (53) sur l'ensemble de liaison d'aiguille-poussoir (5) dans un état initial, et l'engrenage sectoriel (42) sur l'arbre rotatif (41) de la roue rotative (4) correspondant à la seconde crémaillère (63) sur l'ensemble de liaison de rétraction (6) est situé sur la partie supérieure de l'arbre rotatif (41) dans l'état initial.

4. Dispositif de délivrance d'implant oculaire selon la revendication 2, dans lequel la première crémaillère sur l'ensemble de liaison d'aiguille-poussoir (5) est disposée sous les arbres rotatifs (41) de la roue rotative (4), et la seconde crémaillère (63) sur l'ensemble de liaison de rétraction (6) est disposée au-dessus des arbres rotatifs (41) de la roue rotative (4) ; et
l'engrenage sectoriel (42) sur l'arbre rotatif (41) de la roue rotative (4) correspondant à la première crémaillère (53) sur l'ensemble de liaison d'aiguille-poussoir (5) s'engrène avec la première crémaillère (53) sur l'ensemble de liaison d'aiguille-poussoir (5) dans un état initial, et l'engrenage sectoriel (42) sur l'arbre rotatif (41) de la roue rotative (4) correspondant à la seconde crémaillère (63) sur l'ensemble de liaison de rétraction (6) est situé sur la partie inférieure de l'arbre rotatif (41) dans l'état initial.

5. Dispositif de délivrance d'implant oculaire selon la revendication 1, dans lequel l'ensemble de liaison d'aiguille-poussoir (5) comprend l'aiguille-poussoir (51), un élément de liaison d'aiguille-poussoir (52) et la première crémaillère (53) ; et
une extrémité proximale de l'aiguille-poussoir (51) est reliée de manière fixe à une extrémité distale de l'élément de liaison d'aiguille-poussoir (52), une extrémité proximale de l'élément de liaison d'aiguille-poussoir (52) est reliée de manière fixe à la première crémaillère (53) disposée horizontalement, et des dents (43) de la première crémaillère (53) sont disposées vers l'arbre rotatif (41).

6. Dispositif de délivrance d'implant oculaire selon la revendication 1, dans lequel l'ensemble de liaison de rétraction (6) comprend un connecteur (61), un élément de liaison de rétraction (62) et une seconde crémaillère (63) disposée horizontalement, le connecteur (61) étant relié de manière fixe à une extrémité distale de l'élément de liaison de rétraction (62), la seconde crémaillère (63) étant reliée de manière fixe à l'extrémité proximale de l'élément de liaison de rétraction (62), et des dents (43) de la seconde crémaillère (63) étant disposées vers l'arbre rotatif (41).

7. Dispositif de délivrance d'un implant intraoculaire selon la revendication 1, comprenant en outre un siège d'arrêt (64) configuré pour définir une distance entre l'ensemble de liaison d'aiguille-poussoir (5) et l'ensemble de liaison de rétraction (6).

8. Dispositif de délivrance d'un implant intraoculaire selon la revendication 7, dans lequel le siège d'arrêt (64) comprend une première section d'extension et une seconde section d'extension se croisant l'une avec l'autre ;
la première section d'extension est reliée de manière fixe à la seconde section d'extension et est reliée de manière fixe à l'ensemble de liaison de rétractation (6) ou à l'ensemble de liaison d'aiguille-poussoir (5), et s'étend à partir de l'ensemble de liaison de rétractation (6) vers l'ensemble de liaison d'aiguille-poussoir (5) d'une distance prédéterminée ; et
la seconde section d'extension s'étend à partir de la première section d'extension dans une direction s'éloignant de la première section d'extension, et une projection orthogonale de la seconde section d'extension se situe dans une plage de l'ensemble de liaison de rétractation (6) et de l'ensemble de liaison d'aiguille-poussoir (5) selon une direction d'extension de la première section d'extension.

9. Dispositif de délivrance d'implant oculaire selon la revendication 1, dans lequel une extrémité proximale de la gaine (2) est reliée de manière fixe à un siège de gaine qui est disposé dans le boîtier (1) à une position proche du trou traversant, et la gaine (2) s'étend à travers le trou traversant sur le boîtier (1) vers l'extérieur du boîtier (1).

10. Dispositif de délivrance d'implant oculaire selon la revendication 1, dans lequel l'aiguille à ponction (3) est pourvue d'un siège d'aiguille à ponction (31) à une extrémité proximale, l'aiguille à ponction (3) étant reliée de manière fixe au siège d'aiguille à ponction (31) et passant à travers la gaine (2), et le siège d'aiguille à ponction (31) étant relié de manière fixe à une extrémité distale de l'élément de liaison de rétraction (6).

11. Dispositif de délivrance d'implant oculaire selon la revendication 1, dans lequel le boîtier (1) comprend un boîtier supérieur et un boîtier inférieur relié de manière fixe au boîtier supérieur, le boîtier supérieur et le boîtier inférieur comprenant chacun une partie cylindrique (11), une partie de préhension (12) et une partie conique (13) qui sont reliées de manière fixe ensemble ;
un support de montage de roue rotative (17) est disposé sur une surface de paroi interne de la partie cylindrique (11) du boîtier (1), et la roue rotative (4) est montée sur le support de montage de roue rotative (17) par l'intermédiaire des arbres rotatifs (41) ; et
un bloc d'arrêt (16) est prévu sur une surface de paroi interne d'au moins l'une parmi la partie cylindrique (11), la partie de préhension (12) et la partie conique (13), et le bloc d'arrêt (16) est configuré pour limiter l'ensemble de liaison d'aiguille-poussoir (5) et l'ensemble de liaison de rétraction (6) dans une direction radiale.

12. Dispositif de délivrance d'implant oculaire selon la revendication 1, dans lequel la roue rotative (4) est pourvue de dents (43) sur une surface circonférentielle externe, et le boîtier (1) est pourvu d'une gaine coulissante opérationnelle (7) à une position correspondant à l'ouverture (15), la gaine coulissante opérationnelle (7) étant montée de manière coulissante sur le boîtier (1) ; et
la gaine coulissante opérationnelle (7) est pourvue d'une partie de crémaillère (71) sur une paroi interne, la partie de crémaillère (71) s'engrenant avec les dents (43) de la roue rotative (4).

13. Dispositif de délivrance d'implant oculaire selon la revendication 12, dans lequel le boîtier (1) est pourvu d'une gaine à ressort (72) sur une paroi externe, la gaine à ressort (72) s'étendant vers l'extérieur du boîtier (1) et coopérant avec la partie de crémaillère (71) sur une paroi interne de la gaine coulissante opérationnelle (7).
